# EUROPEAN PATENT APPLICATION

(11) **EP 3 583 886 A1**
(43) Date of publication of application: **25.12.2019**
(21) Application number: 18305774.4
(22) Date of filing: 19.06.2018
(51) Int. Cl.: A61B 3/00, A61B 3/024, A61B 3/113, A61K 48/00, G06T 7/13

(54) **ACCURATE OPTICAL DEVICES FOR AGE-RELATED MACULAR DEGENERATION**

(71) Applicant: Nokia Technologies Oy, 02610 Espoo (FI)
(72) Inventor: GARREAU, Alexandre, 92160 Antony (FR); DELAGE, Sylvain, 91400 Orsay (FR)
(74) Representative: Novagraaf Technologies

(57) **Abstract**

An accurate optical device (300) for generating and projecting an intensity encoded artificial image (IEAI) and an outline artificial image (OAI) on an eye having age-macular degeneration treated by receiving optogenetic technique with the incorporation of a first and second type of light sensitive proteins respectively on the On and Off ganglion cells. The device (300) comprises:
• a camera (310) configured to captured a scene viewed by the eye,
• a processing unit (320) configure to generate the artificial images (IEAI, OAI) based on the captured scene (CS); and
• a first and second array of light sources (330a, 330b) respectively configured to reproduce and project alternatively on the eye the intensity encoded artificial image (IEAI) and an outline artificial image (OAI) so as to stimulate the first and second type of light sensitive proteins injected and thus, thanks to the different information encoded, enable to restore a quite accurate vision in the defective area of the eye.

## Description

### Technical Field

Various example embodiments relate to optical devices and associated binoculars for age related macular degeneration.

### Background

The retina is an inner coat of the eye which is a light-sensitive layer of tissue. Photoreceptors are light-sensing cells of the retina, while other cells, like the retinal ganglion cells process photoreceptor signals and send information to the brain via the optic nerve. There are two types of photoreceptors: cones and rods. The entire retina contains about 7 million cones, functioning mainly in daylight and for perception of color, and 75 to 150 million rods, functioning mainly in dim light and for black and white vision.

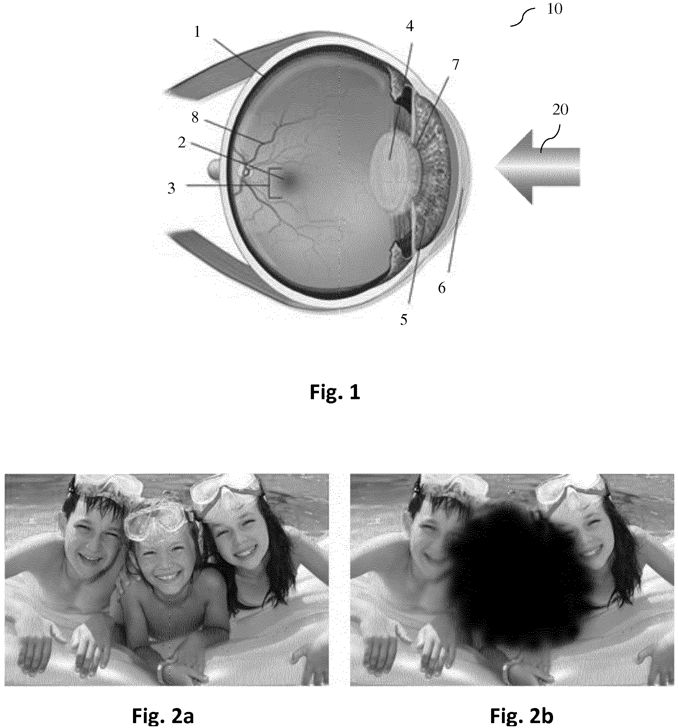

As shown in **Figure 1****,** the retina 1 includes different regions, including the foveola which is located within a region called the macula 3 (6mm in diameter), a yellow section of the human retina. The foveola is approximately 0.35mm in diameter and lies in the center of the fovea 2 (1.5mm in diameter) and contains only cone cells. In this region the cone receptors are found to be longer, slimmer and more densely packed than anywhere else in the retina. It allows that region to have the highest visual acuity.

When photoreceptors degenerate due to disease like retinitis pigmentosa or age-related macular degeneration, the retina can no longer respond to light. However, the other nerve cells of the retina remain in sufficient numbers that electrical stimulation of these cells results in the perception of light.

As shown in **Figure 2a****,** vision of an healthy eye and in **Figure 2b** vision of an eye with age-related macular degeneration, the age-related macular degeneration is characterized by a loss of the vision in the central part of the visual field.

Currently two approaches are studied to compensate this acuity loss and treat persons who suffer retinal degenerative conditions: one based on retinal prostheses, the other one based on optogenetics.

### Retinal prostheses

Retinal prostheses consist of an array of electrodes that are activated, either by light entering the eye in the case of a subretinal prosthesis or by a signal from a camera mounted outside the eye in the case of an epiretinal prosthesis.

The epiretinal approach involves sticking an electronic implant in front of the retina to stimulate the nerve cells. The surgery to do this is rather straightforward, but the epiretinal implant is not itself light sensitive and needs to be connected to a camera outside the eye. It requires an "encoder", whose function is to take over the role of the neurons of the inner retina, which carry out preliminary processing of visual information. Models have been developed for predicting visual sensitivity of patients with epiretinal prosthesis. Different technologies can be used, usually based on silicon devices. Argus II is the reference epiretinal prosthesis with 60 electrodes manufactured by Second Sight Medical Products Inc. It has been already tested on patients all over the world and has been approved by the United States FDA on 14 February 2013.

The subretinal prosthesis replaces the photoreceptors and is placed underneath the retina, which is technically challenging but stimulates the nerve cells in a more natural position. It converts incident light into an electrical signal which is transmitted on to the bipolar cells. Retina is the reference subretinal prosthesis manufactured by Retina Implant AG. It is itself light sensitive, with a 1500 pixel array and does not need an external camera.

The retinal prosthesis is the better advance solution, but presents several drawbacks.

Firstly, the technology based on silicon semiconductor is simple and well known, but this material is always opaque in the visible optical range.

Secondly, the surgery and the implantation of the prosthesis are located to a very delicate zone of the retina.

To generate a defined image on the foveola or the macula, it is necessary to use very low-dimension devices. At the time being, the electronic devices have dimension around 70µm and should be lower around 10µm to reach ideally 2.5µm, corresponding to a cone size for an accurate vision. This size diminish is associated to an increase of electronic devices and so associated with an increase of electric power hardly evacuated in the case of an internal prosthesis.

### Optogenetics

The optogenetic technique modifies individual neurons to incorporate light-sensitive protein (Channelrhodopsin-2 or halorhodopsin) into the cell membrane. By making each cell light sensitive, vision can potentially be restored to near-normal acuity.

However, artificial vision based on optogenetics has its own challenge: modified cells require bright, blue light (460 nm) to be activated, roughly 7 orders of magnitude above the light sensitivity threshold in normally sighted people. Furthermore, it is well-known that blue light has toxic effects on the cells.

There is a real need of an optical device that solves the prior art drawbacks.

### Summary

In a first example embodiment, an accurate optical device is provided for generating and projecting an intensity encoded artificial image and an outline artificial image alternatively on an eye comprising a pupil and a retina, the retina having a field of view comprising at least one defective area, the at least one defective area comprising On and Off ganglion cells having received optogenetic technique with the incorporation of a first type of light sensitive proteins on the On ganglion cells and a second type of light sensitive proteins on the Off ganglion cells. The accurate optical device comprises:
- at least one camera configured to captured a scene viewed in the field of view of the retina;
- a processing unit configured to receive the captured scene, to process edge detection on the captured scene and generate the outline artificial image comprising the detected edges of the captured scene, and to generate the intensity encoded artificial image based on the captured scene, the intensity encoded artificial image being coded in a grayscale; and
- a first array of light sources emitting at a first wavelength configured to stimulate the first type of light sensitive proteins;
- a second array of light sources emitting at a second wavelength configured to stimulate the second type of light sensitive proteins;
wherein the first array of light sources is configured to reproduce the grayscale of the intensity encoded artificial image and to project the reproduced intensity encoded artificial image on the at least one defective area of the retina;
wherein the second array of light sources is configured to reproduce the outline artificial image and to project the reproduced outline artificial image on the at least one defective area of the retina; and
wherein the first and second arrays of light sources emit alternatively

Specific features or embodiments of the accurate optical device, usable alone or in combination (provided that there is no conflict between the different elements) are:
the first and second arrays of light sources are formed on a same semiconductor having active layers with a periodic structure of quantum wells or quantum dots generating alternatively the light sources emitting at the first wavelength and the light sources emitting at the second wavelength, each quantum wells or quantum dots being electrically separated from each other;
the light sources of the first array are electrically polarized oppositely to the light sources of the second array so that one signal is configured to pilot alternatively the light sources of the first and of the second array;
the first wavelength is orange when the first type of light sensitive proteins is channelrhodopsin ReaChR, red when the first type of light sensitive proteins is channelrhodopsin ReaChR and/or Chrimson, or green when the first type of light sensitive proteins is channelrhodopsin ChRGR and/or C1V1, and the second wavelength is yellow when the second type of light sensitive proteins is halorhodopsin;
the first and second arrays of light sources are composed of elements which are transparent at visible wavelength so that the scene viewed by the field of view of the retina apart from the at least one defective area be superimposed respectively with the reproduce intensity encoded artificial image projected by the first array of light sources, and with the reproduced outline artificial image projected by the second array of light sources; and/or
the accurate optical further comprises a first and a second servo control unit, and a pupil motion detection unit configured to detect the motion of the pupil, the pupil motion detection unit being linked to the first and the second servo control unit; wherein the first servo control unit is configured to move the at least one camera according to the pupil motion detection unit; and wherein the second servo control unit is configured to move the set formed by the first and second arrays of light sources according to the pupil motion detection unit.

Other various example embodiments propose a binocular device for a person having a first and a second eye, each eye comprising a pupil and a retina, each retina having a field of view comprising at least one defective area, each at least one defective area comprising On and Off ganglion cells having received optogenetic technique with the incorporation of a first type of light sensitive proteins on the On ganglion cells and a second type of light sensitive proteins on the Off ganglion cells, the binocular device comprising: at least a first accurate optical device as previously described, wherein the first accurate optical device is configured to generate and project a first intensity encoded artificial image and a first outline artificial image alternatively on the first eye.

Specific features or embodiments of the binocular device, usable alone or in combination (provided that there is no conflict between the different elements) are:
the binocular device further comprises a second accurate optical device as previously described, wherein the second accurate optical device is configured to generate and project a second intensity encoded image and a second outline artificial image alternatively on the second eye; and/or
the binocular device is a pair of glasses.

### Brief Description of the Figures

Some embodiments are now described, by way of example only, and with reference to the accompanying drawings, in which :
- Figure 1 schematically illustrates the anatomy of a human eye;
- Figure 2a schematically illustrates the vision of an healthy eye and Figure 2b schematically illustrates the same scene visualized by an eye suffering age-related macular degeneration;
- Figure 3 schematically illustrates an accurate optical device according to a first example embodiment;
- Figure 4 schematically illustrates a diagram a retinal rod/cone pathway;
- Figure 5a schematically illustrates the vision of an eye suffering age-related macular degeneration on which is projected an intensity encoded artificial image superimposed with the normal lateral vision;
- Figure 5b schematically illustrates the vision of an eye suffering age-related macular degeneration on which is projected an outline artificial image superimposed with the normal lateral vision;
- Figure 6 schematically illustrates a first example embodiment of a Vertical Cavity Surface Emitting Laser;
- Figure 7 schematically illustrates a second example embodiment of a Vertical Cavity Surface Emitting Laser coupled to a microlens;
- Figures 8 and 9 schematically illustrate respectively the side and top views of a first and a second array of light sources formed on a same semiconductor;
- Figure 10 schematically illustrates an electrical assembly of a semiconductor as the one of figures 8 and 9;
- Figure 11 schematically illustrates type of power signal applicable to the electrical assembly of figure 10; and
- The Figure 12 schematically illustrates a flowchart of a method for configuring an accurate optical device.

### Description of Embodiments

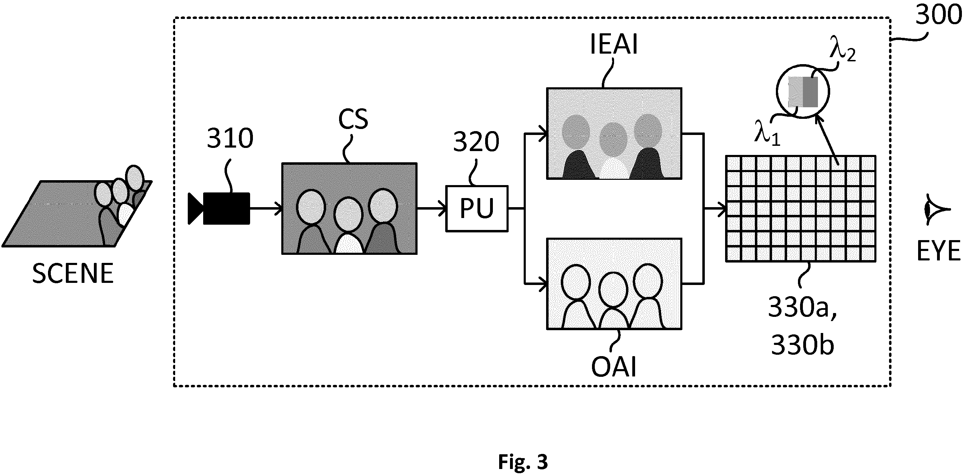

In reference to **Figure 3****,** a first example embodiment provides an accurate optical device 300 for generating and projecting an intensity encoded artificial image IEAI and an outline artificial image OAI alternatively on an eye comprising a pupil and a retina, the retina having a field of view comprising at least one defective area, the at least one defective area comprising On and Off ganglion cells having received optogenetic technique with the incorporation of a first type of light sensitive proteins on the On ganglion cells and a second type of light sensitive proteins on the Off ganglion cells.

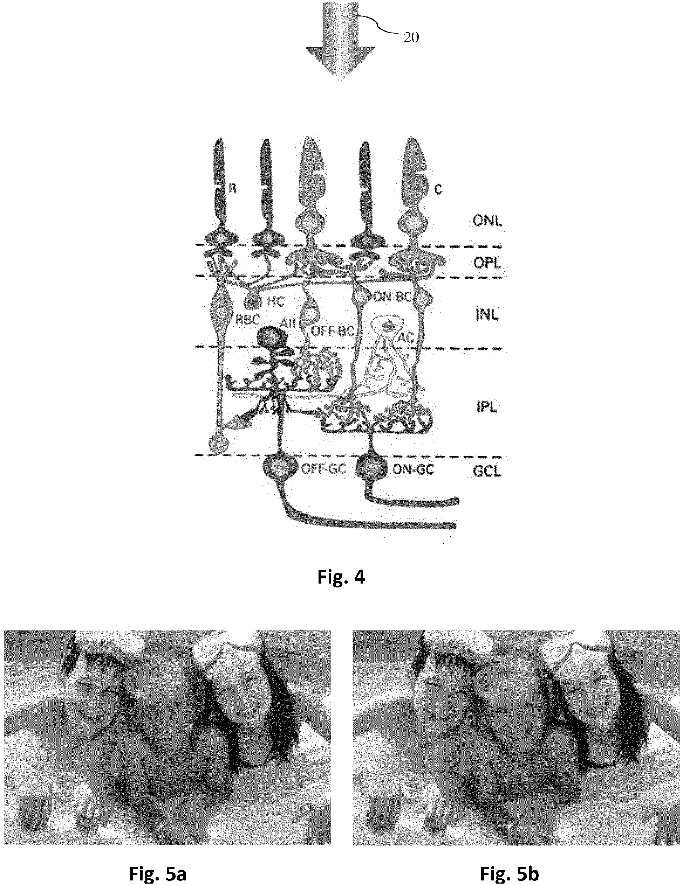

**Figure 4** is a schematic diagram illustrating the retinal rod R / cone C pathway. Multiple layers are superimposed, respectively, in the direction of the scene towards the back of the retina:
- the outer nuclear layer ONL comprising the rod R and cone C photoreceptor cells;
- the outer plexiform layer OPL comprising:
   ∘ the synapses of the rod R photoreceptor cells and the synapses of the rod bipolar cells RBC, of the horizontal cells HC and of the On cone bipolar cells ON-BC to which they are connected; and
   ∘ the synapses of the cone C photoreceptor cells and the synapses of the On cone bipolar cells ON-BC and the Off cone bipolar cells OFF-BC to which they are connected;
- the inner nuclear layer INL comprising the rod R bipolar cells, the On cone bipolar cells ON-BC, the Off cone bipolar cells OFF-BC and the All and AC amancrine cells;
- the inner plexiform IPL comprising:
   ∘ the synapses of the rod R bipolar cells, the All and AC amancrine cells connected to the synapses of the On ganglion cells ON-GC and of the Off ganglion cells OFF-GC;
   ∘ the synapses of the On cone bipolar cells ON-BC, the Off cone bipolar cells OFF-BC of the cones, the All and AC amancrine cells connected to the synapses of the On ganglion cells ON-GC and of the Off ganglion cells OFF-GC; and
- the ganglion cell layer GCL comprising the On ganglion cells ON-GC and the Off ganglion cells OFF-GC.

Examples of first type of light sensitive proteins to be injected on the On ganglion cells are the group of channelrhodopsin like:
- ChR1 and ChR2 which are sensitive to blue light;
- ChRGR and C1V1 which are sensitive to green light;
- ReaChR which are sensitive to orange-red light; or
- Chrimson which are sensitive to red light.

Examples of second type of light sensitive proteins to be injected on the Off ganglion cells are the group of halorhodopsin NpHR sensitive to yellow light.

In relation to **Figure 3**, the accurate optical device 300 comprises:
at least one camera 310;
a processing unit 320
a first array of light sources 330a; and
a second array of light sources 330b.

The at least on camera is configured to captured a scene viewed in a portion of the field of view of the retina comprising the at least one defective area.

The processing unit is configured to:
- receive the captured scene CS;
- process edge detection on the captured scene CS and generate the outline artificial image OAI comprising the detected edges of the captured scene CS; and
- generate the intensity encoded artificial image IEAI based on the captured scene CS, the intensity encoded artificial image IEAI being coded in a grayscale.

After these image processings, the intensity encoded artificial image IEAI and the outline artificial image OAI are respectively send to the first and second array of light sources 330a and 330b.

The first array of light sources 330a comprises sources that emit at a first wavelength λ₁, the first wavelength λ₁ being configured to stimulate the first type of light sensitive proteins. Furthermore, the first array of light sources 330a is configured to reproduce the grayscale of the intensity encoded artificial image IEAI and to project the reproduced intensity encoded artificial image on the at least one defective area of the retina.

The second array of light sources 330b comprises sources that emit at a second wavelength λ₂, the second wavelength λ₂ being configured to stimulate the second type of light sensitive proteins. Furthermore, the second array of light sources is configured to reproduce the outline artificial image OAI and to project the reproduced outline artificial image on the at least one defective area of the retina.

Due to the fact that the first and second array of light sources 330a and 330b both cover the at least one defective area of the retina and, that for a same point of the retina there cannot be a simultaneous stimulation of the On and Off ganglions, the first and second arrays of light sources 330a and 330b emit alternatively.

Furthermore, the projection of two different artificial images comprising different information encoded enables to restore a quite accurate vision in the defective area of the eye. In fact, with the persistence of vision, instead of viewing two different images, humain brain will merge the information of both images and only discern one artificial image of higher resolution / quality. In the same way, humain brain will merge the artificial image of higher resolution projected on the at least one defective area with the lateral (i.e. peripheral) vision see by the rest, i.e. the healthy / normal part, of the retina.

**Figure 5a** illustrates an example of the vision of the eye on which is projected the intensity encoded artificial image IEAI superimposed with the normal lateral vision which correspond to the healthy area of the retina. In this situation, the first array of light sources 330a, which stimulates the On ganglion cells via the first type of light sensitive protein, is configured to reproduce the grayscale of the intensity encoded artificial image IEAI, and to project the reproduced intensity encoded artificial image on the at least one defective area of the retina. Thus, bright color areas in the scene are reproduced with light sources emitting with a high intensity and reciprocally, deep or dark color areas in the scene are reproduced with light sources emitting at lower intensity.

**Figure 5b** illustrates an example of the vision of the eye on which is projected the outline artificial image OAI superimposed with the normal lateral vision which correspond to the healthy area of the retina. In this situation, the second array of light sources 330b, which stimulates the Off ganglion cells via the second type of light sensitive protein, is configured to reproduce the outline artificial image OAI and to project the reproduced outline artificial image on the at least one defective area of the retina. Thus, the sharp outlines or edges detected in the scene are reproduced with light sources emitting with a high intensity and reciprocally, softer outlines or edges detected in the scene are reproduced with light sources emitting at lower intensity. Thus, the outline artificial image OAI generated by the processing unit 320 to be reproduced by the second array of light sources 330b is like a black and white image of the scene but with contrast inversion or a negative of the captured scene.

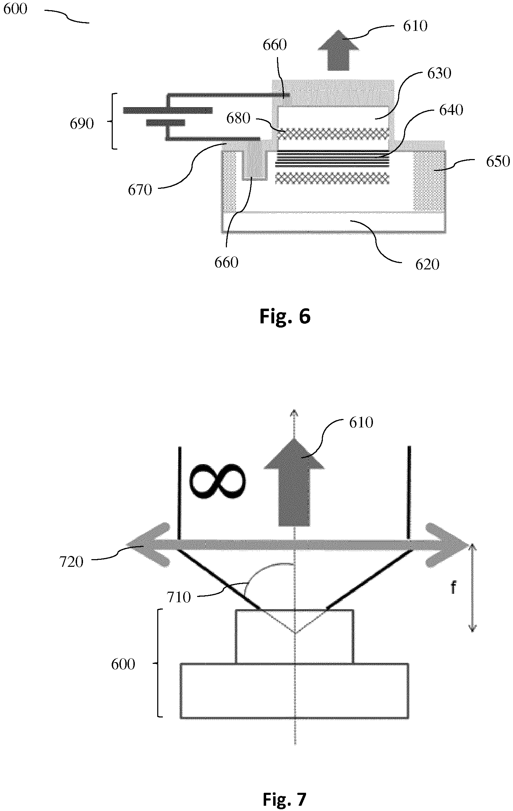

In reference to **Figure 6****,** an example embodiment of a Vertical Cavity Surface Emitting Laser (VCSEL) 600, as a light source for the first or second array of light sources 330a and 330b, emitting a light beam 610 at the corresponding wavelength λ₁ or λ₂.

It is based on one or several epitaxies of InGaN/GaNAs or InGaN/AIGaN multi-quantum wells 640 as intrinsic active layer on a GaN Substrate 620. It is possible to define several multi quantum wells structures 640, by using Selective Area Growth technology (SAG) or Butt-joint technology, in order to obtain the stimulating wavelength which depends on the type of the light-sensitive proteins used (ex: blue, green, yellow, etc.).

On the top of the structure, a GaN layer 630 is grown to finish the epitaxy. If the GaN substrate 620 is doped p, the top epitaxied layers 630 are doped n. Reciprocally, if the GaN substrate 620 is doped n, the top epitaxied layers 630 are doped p, so that the substrate and the top layer form a p-n junction. In order to power the semiconductor diode, the two p and n dopings must be implemented and connected to electrical contacts 660 linked to a power source 690.

The different quantum wells on the same array are electrically separated to prevent electrical interactions or interferences. This isolation can be realized by an insulation layer 650, or by implantation of hydrogen ions H+ or by etching to reach the insulated substrate or layer.

The device can also be isolated to protect it from the environment, by an insulating layer 670 that must be a dielectric material or a semi-insulating semiconductor.

To increase the power of the light emitted, the quantum well generating region 640 can be put between reflecting mirrors 680 forming a laser cavity like two Distributed Bragg Reflectors (DBF).

As illustrated in **Figure 7****,** at the output of the VCSEL 600, the light beam 610 may present a divergence usually associated to the waist 710. So as to avoid interference between the different light beams 610 from other light sources 600, it is advantageous to collimate each light beam 610 emitted by the light source arrays 330a, 330b with a microlens 720 matrix to suppresses this waist 710. The microlens 720 matrix is located a focal distance f of the output of the VCSEL 600.

Additionally, an anti-reflective coating can be deposited on the output of the VCSEL 600 and/or on the microlens 720 matrix so as to keep each light beam generated without optical feedback, which risks disturbing the working of the VCSEL 600.

Advantageously, the first and second arrays of light sources 330a and 330b are formed on a same semiconductor 800 having active layers with a periodic structure of quantum wells or quantum dots generating alternatively the light sources emitting at the first wavelength λ₁ and the light sources emitting at the second wavelength λ₂, each quantum wells or quantum dots being electrically separated from each other.

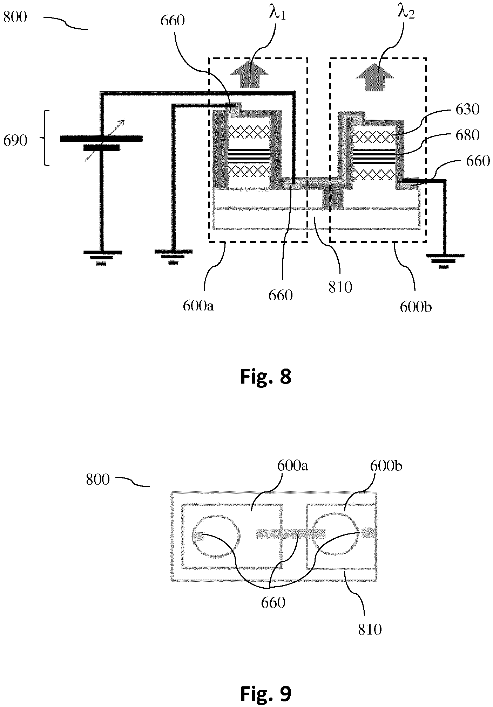

**Figures 8 and 9** illustrate an example embodiment of such a semiconductor 800 comprising schematically a first light source 600a or VCSEL emitting at the first wavelength λ₁ and corresponding to one of the light sources of the first array 330a and a second light source 600b or VCSEL emitting at the second wavelength λ₂ and corresponding to one of the light sources of the second array 330b, on the same insulated substrate 810.

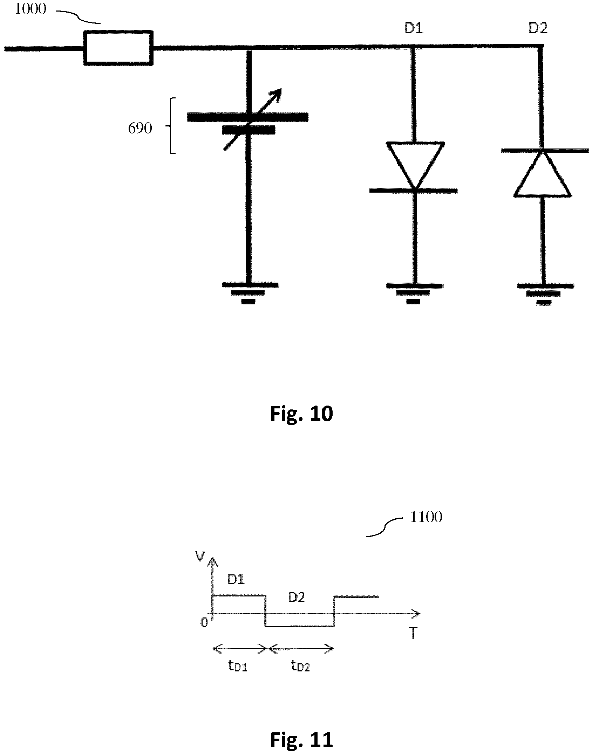

So that one signal be configured to pilot alternatively the light sources of the first and of the second array 330a and 330b, the lights sources of the first array 330a are electrically polarized oppositely to the light sources of the second array 330b. In these **Figures 8 and 9****,** each first and second light source 600a and 600b has a common p (or n) substrate and a common n (or p) top layer 660 forming a p-n junction. So as to polarized the first and second arrays of light sources 330a and 330b, the negative electrical contact 660 of the first light source 600a is connected to the positive electrical contact 660 of the second light source 600b (or reciprocally). An example of such an electrical assembly 1000 is illustrated in **Figure 10** where D1 represents the first light source 600a and D2 represents the second light source 600b.

**Figure 11** illustrates a type of power signal 1100, here a square wave, which can be applied to the electrical assembly 1000. During t_{D1}, a positive voltage is applied to the electrical assembly 1000 so that the first light source D1 emits a light pulse at λ₁ during t_{D1} while the second light source D2 is inhibited. Then a negative voltage is applied so that the second light source D2 emits a light pulse at λ₂ during t_{D2} while the first light source D1 is inhibited, etc. so that the light sources generate alternatively periodical pulses.

Regarding the selection of the first and second wavelengths λ₁ and λ₂ emitted:
- the first wavelength λ₁ is preferably:
   ∘ orange, i.e. between 590 nm and 610 nm when the first type of light sensitive proteins is channelrhodopsin ReaChR;
   ∘ red, i.e. between 610 nm and 760 nm when the first type of light sensitive proteins is channelrhodopsin ReaChR and/or Chrimson; or
   ∘ or green, i.e. between 500 nm and 570 nm when the first type of light sensitive proteins is channelrhodopsin ChRGR and/or C1V1; and
- the second wavelength λ₂ is preferably yellow, i.e.; between 570 nm and 590 nm when the second type of light sensitive proteins is halorhodopsin.

Regarding the structural composition of the active layers associated to generate the appropriate color, the table below lists example of semiconductor composition:

| **Color** | **Semiconductors** |
|---|---|
| Red 610nm < λ < 760nm | AlGaAs, GaAsP, AlGaInP |
| Orange 590nm < λ < 610nm | GaAsP, AlGaInP |
| Yellow 570nm < λ < 590nm | GaN, GaAsP, AlGaInP |
| Green 500nm < λ < 570nm | GaN, GaAsP |
| Blue 450nm < λ < 500nm | InGaN |

Preferably, the first and second arrays of light sources 330a and 330b are based on one or several epitaxies of semiconductor multi-quantum wells of III-V semiconductors as intrinsic active layer on the semiconductor substrate 810 such as:
- InGaN/GaNAs or InGaN/AIGaN multi-quantum wells as intrinsic active layer on a GaN, SiC or Al2O3 Substrate for blue-green ChR stimulation and yellow NpHR stimulation; or
- AlGaInAs multi-quantum wells as intrinsic active layer on a GaAs Substrate for red ChR stimulation and yellow NpHR stimulation.

So that the scene viewed by the field of view of the retina apart from the at least one defective area be superimposed respectively with the reproduce intensity encoded artificial image projected by the first array of light sources, and with the reproduced outline artificial image projected by the second array of light sources, the first and second arrays of light sources 330a and 330b are composed of elements which are transparent at visible wavelength, such as GaN which is also a biocompatible material.

In order to detect the motion of the pupil, and thus redirect the projection of the intensity encoded artificial image IEAI and the outline artificial image OAI on the at least one defective area of the retina, the accurate optical device can further comprise:
- a first and a second servo control unit; and
- a pupil motion detection unit configured to detect the motion of the pupil, the pupil motion detection unit being linked to the first and the second servo control unit.

The first servo control unit is configured to move the at least one camera according to the pupil motion detection unit and the second servo control unit is configured to move the set formed by the first and second arrays of light sources according to the pupil motion detection unit.

In a second example embodiment provides a binocular device for a person having a first and a second eyes, each eye comprising a pupil and a retina, each retina having a field of view comprising at least one defective area, each at least one defective area comprising On and Off ganglion cells having received optogenetic technique with the incorporation of a first type of light sensitive proteins on the On ganglion cells and a second type of light sensitive proteins on the Off ganglion cells.

The binocular device comprises at least a first accurate optical device as previously described, wherein the first accurate optical device is configured to generate and project a first intensity encoded artificial image and a first outline artificial image alternatively on the first eye.

So as to enable the volume perception, the binocular device may further comprise a second accurate optical device as previously described, wherein the second accurate optical device is configured to generate and project a second intensity encoded image and a second outline artificial image alternatively on the second eye.

Advantageously, the binocular device is a pair of glasses more easily to manufacture and to implement or wear.

Furthermore, in contrary to retinal prostheses, binocular devices are easier to manufacture due to larger dimension and easier to implement, no surgical operation is required after the optogenetic treatment of the defective retina.

In a third example embodiment a method for configuring an accurate optical device as previously described.

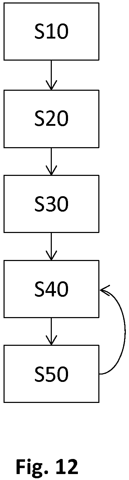

As illustrated in **Figure 12****,** the method comprises five main steps:
a measuring step S10 wherein the field of view of the eye is measured;
a determining step S20 wherein the dimensions of the at least one defective area and the position of the least one defective area in the field of view of the eye are determined;
a configuration step S30 wherein the at least one camera is configured to captured a scene viewed in a portion of the field of view of the retina comprising the at least one defective area;
a processing step S40 wherein the processing unit receives the captured scene, then processes edge detection on the captured scene and generates the outline artificial image comprising the detected edges of the captured scene, and, in parallel, generates the intensity encoded artificial image based on the captured scene, the intensity encoded artificial image being coded in a grayscale; and
a projecting step S50 wherein the first array of light sources is configured to reproduce the grayscale of the intensity encoded artificial image and to project the reproduced intensity encoded artificial image on the at least one defective area of the retina; and wherein the second array of light sources is configured to reproduce the outline artificial image and to project the reproduced outline artificial image on the at least one defective area of the retina; the first and second arrays of light sources emitting alternatively.

The description and drawings merely illustrate the principles of the invention. It will thus be appreciated that those skilled in the art will be able to devise various arrangements that, although not explicitly described or shown herein, embody the principles of the invention and are included within its spirit and scope. Furthermore, all examples recited herein are principally intended expressly to be only for pedagogical purposes to aid the reader in understanding the principles of the invention and the concepts contributed by the inventor(s) to furthering the art, and are to be construed as being without limitation to such specifically recited examples and conditions. Moreover, all statements herein reciting principles, aspects, and embodiments of the invention, as well as specific examples thereof, are intended to encompass equivalents thereof.

### REFERENCES NUMERALS

### Figure 1:

1 Retina
2 Fovea
3 Macula
4 Lens (Crystalline)
5 Iris
6 Cornea
7 Pupil
8 Blood vessels
10 Human eye
20 Light coming from an object and incoming on the eye 10

### Figure 3:

300 Accurate Optical Device
310 Camera
320 Processing Unit
330a First Array of Light Sources
330b Second Array of Light Sources
CS Captured Scene
IEAI Intensity Encoded Artificial Image
OAI Outline Artificial Image

### Figure 4:

All / AC amancrine cells
C Cone photoreceptor cells
GCL Ganglion Cell Layer
HC Horizontal Cell
INL Inner Nuclear Layer
IPL Inner Plexiform
ON-BC On cone Bipolar Cells
ON-GC On Ganglion Cells
ONL Outer Nuclear Layer
OFF-BC Off cone Bipolar Cells
OFF-GC Off Ganglion Cells
OPL Outer Plexiform Layer
R Rod photoreceptor cells
RBC Rod Bipolar Cell

### Figures 6 to 11:

600 Light source / Vertical Cavity Surface Emitting Laser
610 Light Beam
620 substrate
630 Top layer
640 Quantum well amplified region / active layers
650 Insulation
660 Electrical contact
670 Insulation layer
680 Distributed Bragg Reflector
690 Power source
710 Divergence / Waist of the Light Beam
720 Microlens
800 Semiconductor
810 Semiconductor's Substrate
1000 Electrical Assembly
1100 Power Signal
f Focal Distance of the Microlens

## Claims

1. An accurate optical device (300) for generating and projecting an intensity encoded artificial image (IEAI) and an outline artificial image (OAI) alternatively on an eye comprising a pupil and a retina, the retina having a field of view comprising at least one defective area, the at least one defective area comprising On and Off ganglion cells having received optogenetic technique with the incorporation of a first type of light sensitive proteins on the On ganglion cells and a second type of light sensitive proteins on the Off ganglion cells, the accurate optical device (300) comprising:
• at least one camera (310) configured to captured a scene viewed in a portion of the field of view of the retina comprising the at least one defective area ;
• a processing unit (320) configured to receive the captured scene (CS), to process edge detection on the captured scene (CS) and generate the outline artificial image (OAI) comprising the detected edges of the captured scene (CS), and to generate the intensity encoded artificial image (IEAI) based on the captured scene (CS), the intensity encoded artificial image (IEAI) being coded in a grayscale; and
• a first array of light sources (330a) emitting at a first wavelength λ₁ configured to stimulate the first type of light sensitive proteins;
• a second array of light sources emitting (330b) at a second wavelength λ₂ configured to stimulate the second type of light sensitive proteins;
wherein the first array of light sources (330a) is configured to reproduce the grayscale of the intensity encoded artificial image (IEAI) and to project the reproduced intensity encoded artificial image (IEAI) on the at least one defective area of the retina;
wherein the second array of light sources (330b) is configured to reproduce the outline artificial image (OAI) and to project the reproduced outline artificial image (OAI) on the at least one defective area of the retina; and
wherein the first and second arrays of light sources (330a, 330b) emit alternatively.

2. The accurate optical device (300) according to claim 1 wherein the first and second arrays of light sources (330a, 330b) are formed on a same semiconductor (800) having active layers with a periodic structure of quantum wells or quantum dots (640) generating alternatively the light sources (600a) emitting at the first wavelength λ₁ and the light sources (600b) emitting at the second wavelength λ₂, each quantum wells or quantum dots being electrically separated from each other.

3. The accurate optical device (300) according to any one of claims 1 to 2 wherein the light sources (600a) of the first array (330a) are electrically polarized oppositely to the light sources (600b) of the second array (330b) so that one signal (1100) is configured to pilot alternatively the light sources (600a, 600b) of the first and of the second arrays (330a, 330b).

4. The accurate optical device (300) according to any one of claims 1 to 3 wherein the first wavelength λ₁ is orange when the first type of light sensitive proteins is channelrhodopsin ReaChR, red when the first type of light sensitive proteins is channelrhodopsin ReaChR and/or Chrimson, or green when the first type of light sensitive proteins is channelrhodopsin ChRGR and/or C1V1, and wherein the second wavelength λ₂ is yellow when the second type of light sensitive proteins is halorhodopsin.

5. The accurate optical device (300) according to anyone of claims 1 to 4 wherein, the first and second arrays of light sources (330a, 330b) are composed of elements which are transparent at visible wavelength so that the scene viewed by the field of view of the retina apart from the at least one defective area be superimposed respectively with the reproduce intensity encoded artificial image (IEAI) projected by the first array of light sources (330a), and with the reproduced outline artificial image (OAI) projected by the second array of light sources (330b).

6. The accurate optical device (300) according to any one of claims 1 to 5 further comprising:
• a first and a second servo control unit; and
• a pupil motion detection unit configured to detect the motion of the pupil, the pupil motion detection unit being linked to the first and the second servo control unit;
wherein the first servo control unit is configured to move the at least one camera (310) according to the pupil motion detection unit; and
wherein the second servo control unit is configured to move the set formed by the first and second arrays of light sources (330a, 330b) according to the pupil motion detection unit.

7. A binocular device for a person having a first and a second eyes, each eye comprising a pupil and a retina, each retina having a field of view comprising at least one defective area, each at least one defective area comprising On and Off ganglion cells having received optogenetic technique with the incorporation of a first type of light sensitive proteins on the On ganglion cells and a second type of light sensitive proteins on the Off ganglion cells, the binocular device comprising: at least a first accurate optical device according to any one of the claims 1 to 6, wherein the first accurate optical device is configured to generate and project a first intensity encoded artificial image and a first outline artificial image alternatively on the first eye.

8. The binocular device of claim 7 further comprising a second accurate optical device according to any one of the claims 1 to 6, wherein the second accurate optical device is configured to generate and project a second intensity encoded artificial image and a second outline artificial image alternatively on the second eye.

9. The Binocular device as claim in any one of claims 7 to 8, wherein the binocular device is a pair of glasses.

10. A method for configuring an accurate optical device (300) as claimed in anyone of claims 1 to 6, the method comprising:
• a measuring step wherein the field of view of the eye is measured;
• a determining step wherein the dimensions of the at least one defective area and the position of the least one defective area in the field of view of the eye are determined;
• a configuration step wherein the at least one camera (310) is configured to captured a scene viewed in a portion of the field of view of the retina comprising the at least one defective area;
• a processing step wherein the processing unit (320) receive the captured scene (CS), then process edge detection on the captured scene (CS) and generate the outline artificial image (OAI) comprising the detected edges of the captured scene (CS), and, in parallel, generate the intensity encoded artificial image (IEAI) based on the captured scene (CS), the intensity encoded artificial image (IEAI) being coded in a grayscale; and
• a projecting step wherein the first array of light sources (330a) is configured to reproduce the grayscale of the intensity encoded artificial image (IEAI) and to project the reproduced intensity encoded artificial image (IEAI) on the at least one defective area of the retina; and wherein the second array of light sources is configured to reproduce the outline artificial image (OAI) and to project the reproduced outline artificial image (OAI) on the at least one defective area of the retina; the first and second arrays of light sources emitting alternatively.
